# EUROPÄISCHE PATENTSCHRIFT

(11) **EP 4 228 543 B1**
(45) Veröffentlichungstag und Bekanntmachung des Hinweises auf die Patenterteilung: **14.08.2024**
(21) Anmeldenummer: 22813170.2
(22) Anmeldetag: 28.10.2022
(51) Int. Cl.: A61B 34/20, A61B 90/20, A61B 90/96

(54) **CHIRURGISCHES NAVIGATIONSSYSTEM MIT VERBESSERTEM INSTRUMENTENTRACKING UND NAVIGATIONSVERFAHREN**
SURGICAL NAVIGATION SYSTEM HAVING IMPROVED INSTRUMENT TRACKING AND NAVIGATION METHOD
SYSTÈME DE NAVIGATION CHIRURGICALE AYANT UN SUIVI D'INSTRUMENT AMÉLIORÉ ET PROCÉDÉ DE NAVIGATION

(30) Priorität: 02.11.2021 DE 102021128478
(43) Veröffentlichungstag der Anmeldung: 23.08.2023
(73) Patentinhaber: B. Braun New Ventures GmbH, 79110 Freiburg im Breisgau (DE)
(72) Erfinder: STAWIASKI, Jean, 79199 Kirchzarten (DE); SARVESTANI, Amir, 79104 Freiburg (DE)
(74) Vertreter: Winter, Brandl - Partnerschaft mbB
(86) Internationale Anmeldenummer: PCT/EP2022/080276
(87) Internationale Veröffentlichungsnummer: WO 2023/078803

(56) Entgegenhaltungen:
- US-A1- 2001 027 272
- US-A1- 2002 151 784
- US-A1- 2008 013 809

## Beschreibung

### Technisches Gebiet

Die vorliegende Offenbarung betrifft ein chirurgisches Navigationssystem zur Navigation bei einem chirurgischen Eingriff bei einem Patienten zur Nachverfolgung/ zum Tracken von zumindest einem medizinischen Instrument, welches bei dem Eingriff eingesetzt wird. Daneben betrifft die vorliegende Offenbarung einen Navigationsturm, ein Navigationsverfahren sowie ein computerlesbares Speichermedium gemäß den Oberbegriffen der nebengeordneten Ansprüche.

### Hintergrund der vorliegenden Offenbarung

Üblicherweise ist eine chirurgische Navigation nur mit besonderen Instrumenten durchführbar, welche spezielle Markierungssysteme mit Markern, wie etwa Infrarot-Referenzpunkte, oder ein elektromagnetisches Tracking-System aufweisen. Diese Instrumente werden als Zeiger oder Pointer bezeichnet und sind speziell dafür angepasst, mit ihrer Spitze eine Position im dreidimensionalen Raum zu markieren, welche durch das Navigationssystem erfasst wird.

Um nun während eines Eingriffs die benötigten Navigationsinformationen zu erhalten, muss ein Nutzer, etwa ein Chirurg, seinen Arbeitsablauf unterbrechen, das speziell angepasste (Pointer-)Instrument in seine Hand nehmen und an die gewünschte Stelle führen, wobei die einzige Funktion des (Pointer-)Instruments die Bereitstellung von Navigationsinformationen ist. Ein derartiger kontinuierlicher Wechsel zwischen den einzelnen Instrumenten verlängert in nachteiliger Weise einen Eingriff bei einem Patienten und führt darüber hinaus zu einer Ermüdung des Chirurgen. Auch ist stets eine Vielzahl von medizinischen Instrumenten mit unterschiedlichen Funktionen bereitzuhalten.

Zudem besteht aktuell das Problem, dass Navigationssysteme üblicherweise ein externes Kamerasystem verwenden, etwa eine Stereokamera, die beabstandet zu einem Eingriffsbereich angeordnet sind. Bei einem chirurgischen Navigationssystem, beispielsweise einem infrarotbasierten Navigationssystem, wird in einer unübersichtlichen Umgebung eines Eingriffsbereichs daher ein Sichtbereich innerhalb von Sichtlinien stark begrenzt, insbesondere wenn ein Navigationssystem und ein Operationsmikroskop gleichzeitig verwendet werden. Die Nachverfolgung des Instruments wird temporär unterbrochen und ebenso verschlechtert sich eine Präzision der Nachverfolgung. Zudem sind Restriktionen hinsichtlich eines freizuhaltenden Sichtbereichs für das Kamerasystem einzuhalten, welche den Eingriff am Patienten weiter erschweren.

Die US 2002/0151784 A1 offenbart ein Assistenzsystem für einen chirurgischen Eingriff unter Verwendung eines Operationsmikroskops. Eine Position des Mikroskops und eines starr an das Mikroskop befestigten Instruments wird dabei mittels LEDs des Mikroskops erfasst, welche durch ein Trackingsystem in Form einer Stereokamera nachverfolgt wird.

Ferner offenbaren die US 2001/0027272 A1 und die US 2008/0013809 A1 Verfahren und Systeme für eine Nachverfolgung.

### Zusammenfassung der Offenbarung

Es sind daher die Aufgaben und Ziele der vorliegenden Offenbarung, ein chirurgisches Navigationssystem, einen Navigationsturm, ein Navigationsverfahren sowie ein computerlesbares Speichermedium zur Verfügung zu stellen, das die Nachteile aus dem Stand der Technik vermeidet oder zumindest mindert. Insbesondere soll ein chirurgisches Navigationssystem sowie Navigationsverfahren bereitgestellt werden, das eine höhere Präzision einer Nachverfolgung/ eines Trackings eines medizinischen Instruments liefert und eine kontinuierliche und unterbrechungsfreie Nachverfolgung sowie eine noch bessere Erfassung eines Eingriffsbereichs erlaubt. Ein Nutzer, insbesondere Chirurg soll eine bessere und sicherere Navigationsmodalität erhalten. Zudem ist eine Teilaufgabe, einen chirurgischen Arbeitsablauf noch besser zu unterstützen und schneller durchzuführen. Eine weitere Teilaufgabe kann darin gesehen werden, bestehende chirurgische Instrumente als Pointer zu nutzen oder zumindest nur geringfügig ändern zu müssen, insbesondere nur ergänzen. Auch ist eine weitere Teilaufgabe, eine zur Verfügung stehende Navigationszeit während eines Eingriffs zu erhöhen.

Die Aufgaben der vorliegenden Offenbarung werden hinsichtlich eines gattungsgemäßen chirurgischen Navigationssystems erfindungsgemäß durch die Merkmale des Anspruchs 1 gelöst, hinsichtlich eines gattungsgemäßen Navigationsturms erfindungsgemäß durch die Merkmale des Anspruchs 9, hinsichtlich eines Navigationsverfahrens erfindungsgemäß durch die Merkmale des Anspruchs 10 und hinsichtlich eines computerlesbaren Speichermediums erfindungsgemäß durch die Merkmale des Anspruchs 14.

Ein Grundgedanke der vorliegenden Offenbarung kann also darin gesehen werden, bei dem chirurgischen Navigationssystem und Navigationsverfahren ein medizinisches Instrument nicht direkt und absolut über etwa ein Kamerasystem zu erfassen, sondern indirekt über einerseits die Nachverfolgung des Aufnahmekopfs der bildgebenden medizinischen Vorrichtung, welche näher am Eingriffsbereich angeordnet ist als etwa das Kamerasystem, und andererseits der Nachverfolgung des medizinischen Instruments durch den Aufnahmekopf selber, so dass über eine serielle Verknüpfung der beiden Nachverfolgungen die Position und/oder Orientierung des Instruments berechenbar bzw. bestimmbar ist. Wenn der Patient gegenüber dem Navigationssystem registriert ist, so sind auch 3D-Aufnahmedaten, insbesondere präoperative 3D-Aufnahmedaten wie MRT-Aufnahmen und/oder CT-Aufnahmen, gegenüber dem Patienten registriert und die bestimmte Position und/oder Orientierung des Instruments kann in den 3D-Aufnahmedaten für eine Navigation bestimmt und dem Nutzer dann dargestellt werden.

Es wird gewissermaßen eine Entkopplung der direkten Nachverfolgung des Instruments mit zumindest zweiteiliger, sequentieller Nachverfolgung vorgesehen, nämlich einmal der Nachverfolgung des Aufnahmekopfs, und einer davon unabhängigen Nachverfolgung des Instruments durch den Aufnahmekopf. Die beiden Nachverfolgungen stellen zwei einzelne (Koordinaten-)Transformationen bereit, die miteinander verknüpft werden, um eine Transformation für das nachzuverfolgende Instrument zu erhalten. Durch die Unabhängigkeit können auch unterschiedliche Nachverfolgungssystem bzw. Methoden eingesetzt werden, so dass zur Nachverfolgung des Aufnahmekopfs ein auf diesen ausgerichtetes und für größere Entfernungen angepasstes Trackingsystem verwendet werden kann, während zur Nachverfolgung des Instruments auch eine unterschiedliche und speziell angepasste Nachverfolgung für einen geringeren Abstand im Bereich des Eingriffs durchführbar ist. Auf diese Weise können sogar übliche, etwa standardisierte medizinische, insbesondere chirurgische, Instrumente erfasst werden, was beim Stand der Technik nicht möglich ist.

Durch diese zumindest gekoppelte/ verknüpfte zweigeteilte bzw. zweistufige, serielle Nachverfolgung kann eine besonders hohe Präzision eines Trackings/ eine hohe Nachverfolgungsgenauigkeit aufgrund des geringen Abstands zwischen (dem Aufnahmekopf) der bildgebenden medizinischen Vorrichtung, insbesondere eines Stereomikroskops, und dem verwendeten Instrument erreicht werden. Ebenso werden auf diese Weise die Probleme mit einer Sichtlinie bei etwa einer externen Navigationskamera vermieden, da die Navigationskamera das nachzuverfolgende Instrument selbst nicht sehen muss, sondern nur den Aufnahmekopf der bildgebenden medizinischen Vorrichtung, insbesondere einen Mikroskopkopf mit optischem System eines Operationsmikroskops. Auch ist von Vorteil, dass der chirurgische Arbeitsablauf durch den Einsatz des Navigationssystems nicht unterbrochen wird, da durch die Erfassung und Nachverfolgung des Instruments durch den Aufnahmekopf der bildgebenden Vorrichtung, auch Standardinstrumente verfolgt werden können und nicht gegen navigierte Instrumente, wie einen Pointer, ausgetauscht werden müssen.

Auch wird ein Eingriff mit Navigationsverfahren noch sicherer, da die Netto-Navigationszeit, welche dem Chirurgen zur Verfügung steht, weiter erhöht wird. Chirurgische Standardinstrumente werden während ihres gesamten Einsatzes navigiert.

Mit anderen Worten wird ein chirurgisches Navigationssystem zur Navigation bei einem chirurgischen Eingriff bei einem Patienten zur Nachverfolgung von zumindest einem Objekt, insbesondere medizinischen Instrument, bereitgestellt, aufweisend: eine Darstellungsvorrichtung, insbesondere einen Monitor, zur Darstellung eines visuellen Inhalts; zumindest ein nachzuverfolgendes Objekt, insbesondere medizinisches Instrument; eine medizinische, bildgebende Vorrichtung mit einem Aufnahmekopf, der dafür angepasst ist, bei einem Patienten eine optische bzw. visuelle Aufnahme eines Eingriffsbereichs zu erstellen sowie in dem chirurgischen Eingriffsbereich des Patienten das nachzuverfolgende Objekt, insbesondere medizinische Instrument, zu erfassen und gegenüber dem Aufnahmekopf nachzuverfolgen/ zu tracken; ein (externes) Trackingsystem/ Nachverfolgungssystem, welches dafür angepasst ist, zumindest den Aufnahmekopf der bildgebenden Vorrichtung zu erfassen und gegenüber dem Trackingsystem nachzuverfolgen, sowie zumindest einen Teilabschnitt des Patienten mit dem Eingriffsbereich für eine Registrierung zu erfassen und insbesondere nachzuverfolgen; eine Datenbereitstellungseinheit, insbesondere eine Speichereinheit, welche dafür angepasst ist, digitale 3D-Aufnahmedaten, insbesondere präoperative 3D-Aufnahmedaten, des Patienten bereitzustellen; und eine Steuereinheit, die dafür angepasst ist, die Daten der bildgebenden Vorrichtung, die Daten des Trackingsystems sowie die bereitgestellten 3D-Aufnahmedaten zu verarbeiten und durch Verknüpfung (einer Transformation bzw. Transformationsmatrix) der Nachverfolgung von dem Trackingsystem zu dem Aufnahmekopf sowie der Nachverfolgung von dem Aufnahmekopf zu dem Objekt, insbesondere Instrument, eine Position und/oder Orientierung des nachzuverfolgenden Objekts, insbesondere Instruments, insbesondere einer Instrumentenspitze des Instruments, zu bestimmen und diese auf die 3D-Aufnahmedaten des zu dem Trackingsystem registrierten Patienten zu übertragen und diese durch die Darstellungsvorrichtung visuell auszugeben. Die Steuereinheit kann eine Korrelationsdarstellung mit den zu dem Patienten registrierten 3D-Aufnahmedaten und dieser bestimmten/ berechneten Position und/oder Orientierung des Instruments erstellen und durch die Darstellungsvorrichtung visuell auszugeben. Auf diese Weise kann der Chirurg auf beispielsweise einem OP-Monitor das (virtuelle) Instrument, insbesondere die genaue Position der Instrumentenspitze, in den 3D-Aufnahmedaten sich anzeigen lassen.

Es kann also auf Basis des erfassten Patienten, des erfassten Aufnahmekopfs und das durch den Aufnahmekopf erfasste Instrument eine Position und/oder Orientierung des Instruments, insbesondere einer Instrumentenspitze, zu den 3D-Aufnahmedaten bestimmt werden. Auf diese Weise wird eine Positions- und/oder Orientierungsbestimmung von chirurgischen Instrumenten relativ zu 3D-Aufnahmedaten (3D-Bild) des Patienten ermöglicht.

Es wird also ein Navigationssystem zur Verfolgung chirurgischer Instrumente und zur Anzeige ihrer Position, insbesondere ihrer Lage, relativ zu 3D-Aufnahmedaten (3D-Bildsatz) des Patienten während der Operation bereitgestellt, wobei das Instrument mit einem optischen System verfolgt wird, das optische System wiederum von einem Trackingsystem des Navigationssystems verfolgt wird und der Patient ebenso von dem Navigationssystem (für eine Registrierung zu den 3D-Aufnahmedaten) verfolgt wird.

Der Begriff "Position" meint eine geometrische Position im dreidimensionalen Raum, der insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an. Man kann auch sagen, dass durch die Orientierung eine Ausrichtung angegeben wird mit Richtungs- bzw. Drehungsangabe im dreidimensionalen Raum. Insbesondere kann die Orientierung mittels drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie drei Winkelkoordinaten für die Orientierung.

Der Begriff 3D definiert dabei, dass die Aufnahmedaten räumlich, also dreidimensional vorliegen. Der Körper des Patienten oder zumindest ein Teilbereich des Körpers mit räumlicher Ausdehnung kann in einem dreidimensionalen Raum mit etwa einem kartesischen Koordinatensystem (X, Y, Z) digital als Aufnahmedaten vorliegen.

Das medizinische nachzuverfolgende Instrument kann beispielsweise ein Saugrohr oder eine Pinzette sein, mit deren distalen Instrumentenspitze sich besonders präzise ein Punkt im Raum definieren lässt. Durch das chirurgische Navigationssystem lassen sich natürlich auch (kleinere) medizinische Geräte nach verfolgen.

Vorteilhafte Ausführungsformen sind in den Unteransprüchen beansprucht und werden insbesondere nachfolgend erläutert.

Gemäß einer bevorzugten Ausführungsform kann die medizinische bildgebende Vorrichtung ein chirurgisches Operationsmikroskop und/oder ein medizinisches Endoskop/ Operationsendoskop sein, das dafür angepasst ist, eine räumliche Erfassung zur Nachverfolgung durchzuführen, und insbesondere ein 3D-Kamerasystem zur Erfassung von Tiefeninformationen aufweist. Beispielsweise in der Kraniotomie unterstützt ein Operationsmikroskop den Chirurgen während des Eingriffs. Die Position des Mikroskopkopfs des Operationsmikroskops wird mit dem Trackingsystem des chirurgischen Navigationssystems verfolgt. Insbesondere wird ein Stereo-Operationsmikroskop wird während des Eingriffs verwendet, um chirurgische Instrumente zu erkennen und ihre relative Position in Bezug auf die Bildsensoren des Mikroskops zu berechnen. Das bildgebende Vorrichtung (Sehsystem) kann also ein Operationsmikroskop oder ein Operationsendoskop sein. Insbesondere kann das chirurgisches Navigationssystem also in Verbindung mit einem Operationsmikroskop, insbesondere einem Stereo-Mikroskop, verwendet werden, wobei die Steuereinheit speziell angepasst ist, entsprechende Navigationsdaten zu liefern. Mit einem (üblichen) Operationsmikroskop kann man einen interessierenden Teil des Eingriffsbereichs bis auf wenige Zentimeter Abstand anvisieren und das Instrument besonders präzise lokalisieren und nach verfolgen.

Gemäß einer weiteren bevorzugten Ausführungsform kann der Aufnahmekopf der medizinischen bildgebenden Vorrichtung eine Stereo-Kamera für eine Stereo-Aufnahme aufweisen und insbesondere die Steuereinheit dafür angepasst sein, mittels maschinellen Sehens/ Machine Vision aus der Stereo-Aufnahme eine räumliche, dreidimensionale Position und/oder Orientierung des Instruments, insbesondere der Instrumentenspitze, gegenüber dem Aufnahmekopf zu erfassen. Mit anderen Worten kann die bildgebende Vorrichtung ein Stereokamerasystem und/oder ein 3D-Kamerasystem mit Tiefeninformationen aufweisen. Es muss so lediglich die Steuereinheit für die Auswertung entsprechend angepasst werden und es können standardisierte Geräte wie etwa Stereo-Mikroskope oder Endoskope mit einer stirnseitigen Stereo-Kamera eingesetzt werden.

Gemäß einem weiteren Ausführungsbeispiel der Offenbarung kann das Navigationssystem eine Bildanalyseeinrichtung aufweisen, die dafür angepasst ist, mittels maschinellen Sehens/ Machine Vision aus zumindest zwei Aufnahmeperspektiven, insbesondere einer Stereo-Aufnahme, eine räumliche dreidimensionale Erfassung eines Instruments durchzuführen. Mit anderen Worten kann das Navigationssystem ein Kamerasystem mit zumindest einer Kamera aufweisen und auf der Basis von maschinellem Sehen eine dreidimensionale Erfassung und Nachverfolgung des Instruments durchführen.

Vorzugsweise kann die Steuereinheit dafür angepasst sein, eine dreidimensionale Position und/oder Orientierung des Instruments mittels Bildverarbeitungs(analyse)techniken und/oder mittels Triangulation der Stereo-Aufnahme! eines Stereobildes und/oder mittels Rekonstruktion einer Disparitätsüberlappung einer Stereo-Aufnahme zu bestimmen. Insbesondere kann die Steuereinheit also dafür angepasst sein, mittels Prinzipien der 3D-Rekonstruktion und Positionsbestimmung aus Stereo-Aufnahmen eine Navigation durchzuführen. Hierbei wird insbesondere für jeden Pixel im linken Bild der Stereo-Aufnahme einen entsprechenden Pixel im rechten Bild gesucht. Die Positionen dieser beiden Pixel werden verwendet, um die scheinbare Tiefe des Pixels zu berechnen. Alternativ oder zusätzlich kann die Lage (3D-Position) eines chirurgischen Instruments zu dem Aufnahmekopf mithilfe von Bildverarbeitungstechniken berechnet werden. Insbesondere kann durch die Erkennung des Instruments im linken und rechten Bild nach dem Prinzip der Triangulation die Lage (3D-Position) des Instruments bestimmt werden. Alternativ oder zusätzlich kann durch Rekonstruktion einer Disparitätsüberlappung der Stereo-Aufnahmen die Position und/oder Orientierung bestimmt werden. In einer solchen Tiefenkarte/ Disparitätskarte wird ein Instrument erkannt und seine Lage (3D-Position) direkt aus den entsprechenden Tiefenwerten errechnet. Die Erkennung von Instrumenten kann dabei mit folgenden Methoden durchgeführt werden: Bildverarbeitungsmethoden für jedes einzelne Farbbild der linken Aufnahme bzw. der rechten Aufnahme; Bildverarbeitungsmethoden, die ein Paar der linken Aufnahme und der rechten Aufnahme (gleichzeitig) verwenden; Bildverarbeitungsmethoden, die eine einzige Disparitätskarte/ Tiefenkarte verwenden; oder auch Bildverarbeitungsmethoden, die eine Kombination der vorgenannten Bildverarbeitungsmethoden verwenden. Insbesondere umfassen die Methoden des maschinellen Sehens auch Deep-Learning-Methoden unter Verwendung von neuronalen Netzen oder Bildtransformationen (Vision Transformers); Handgefertigte Design-Merkmale wie Linienerkennung oder Farberkennung; oder eine Anpassung von 3D-Modellen zu Aufnahmen. Insbesondere wird das zumindest eine Instrument, vorzugsweise durch maschinelles Sehen, durch ein chirurgisches (Stereo-Operations-)Mikroskop nachverfolgt und das Operationsmikroskop selbst wird durch ein Trackingsystem des Navigationssystems nachverfolgt.

Insbesondere kann an einer optisch sichtbaren Außenseite des nachzuverfolgenden medizinischen Instruments, insbesondere an einem distalen Ende oder Endabschnitt des Instruments, ein vorbestimmtes /definiertes optisches Muster vorgesehen, insbesondere integriert, sein, welches der Aufnahmekopf erfasst. Die Steuereinheit ist ihrerseits dafür angepasst ist, das optische Muster zu erkennen und zu entschlüsseln oder mit einer in einer Speichereinheit hinterlegten Referenz zu vergleichen und auf Basis des erfassten optischen Musters eine Position einer Instrumentenspitze relativ zu dem optischen Muster oder eine Geometrie des Instruments zu bestimmen. Über das optische Muster kann die Steuereinheit also Informationen zu einer Position der Instrumentenspitze oder zu einer Geometrie des Instruments erhalten. Das zumindest eine Instrumente hat also ein vordefiniertes optisches Muster/ optische Markierung, das insbesondere in dem distalen Ende oder Endabschnitt integriert ist, und von dem Aufnahmekopf erfasst wird. Die über das optische Muster transportierten Informationen können nützlich sein, da die Erkennung der genauen Position der Instrumentenspitze aufgrund einer Verdeckung der Instrumentenspitze oder bei einem geringen Kontrast des Instruments schwierig ist. Die Erkennung eines bestimmten optischen Musters an einem Hauptkörper-Abschnitt des Instruments ist hingegen deutlich einfacher und mit der Information kann die Steuereinheit auf die Position der Instrumentenspitze schließen. Ein Vorteil besteht darin, dass eine noch bessere Ergonomie einer Navigation bereitgestellt wird. Insbesondere sind die chirurgischen Instrumente, welche bei dem Navigationssystem eingesetzt werden, nicht oder nur geringfügig, insbesondere durch eine Markierung, vorzugsweise an oder im Bereich der Spitze, modifiziert. Diese, relativ gesehen einfache und kleine Modifizierung, wird dahingehend durchgeführt, um das Instrument noch präziser zu verfolgen, im Vergleich zu klassischen navigierten Instrumenten mit großen starren Körpern. Zum Beispiel die direkte optische Markierung erreicht werden durch ein einfaches Gravieren von spezifischen Mustern auf dem Instrument, wie z. B. QR-Codes, Datamatrix-Codes, Barcodes, Linien, Punkte oder Texturen. Bei den optischen Markierungen kann es sich um QR-Codes, zumindest zwei Ringe mit vordefiniertem Abstand zueinander oder ein anderes eindeutiges Muster handeln, welches die Steuereinheit entschlüsseln und insbesondere mit hinterlegten Informationen verknüpfen kann. Diese optischen Markierungen können insbesondere verwendet werden, um die Position der Instrumentenspitze zu kodieren und/oder die Geometrie des Instruments anzugeben. Insbesondere kann das vorbestimmte optische Muster ein QR Code sein und in dem QR Code kann ein Abstand von dem QR-Code zu der Instrumentenspitze kodiert hinterlegt sein, so dass die Position der Instrumentenspritze bestimmbar ist.

Gemäß einer weiteren Ausführungsform kann das optische Muster ein QR-Code sein, wobei in dem QR-Code ein Abstand von dem QR-Code zu der Instrumentenspitze enkodiert hinterlegt ist, so dass die Position der Instrumentenspritze bestimmbar ist.

Insbesondere kann mithilfe von Bildverarbeitungstechniken das visuelle Erscheinungsbild gängiger chirurgischer Instrumente erlernt werden, um eine optimale Erkennungsgenauigkeit zu liefern.

Gemäß einer Ausführungsform kann in einer Speichereinheit eine geometrische Form des zumindest einen medizinischen Instruments, insbesondere von mehreren medizinischen Instrumenten, hinterlegt sein, insbesondere durch eine initiale dreidimensionale Erfassung durch den Aufnahmekopf und/oder das Trackingsystem, und die Steuereinheit auf Basis eines durch den Aufnahmekopf erfassten Teilabschnitts des nachzuverfolgenden Instruments und der gespeicherten geometrischen Struktur die Position, insbesondere Lage, der distalen Spitze bestimmt. Die Erkennung von Instrumenten kann insbesondere dadurch vereinfacht werden, indem das visuelle oder geometrische Erscheinungsbild des zumindest einen Instruments vereinheitlicht wird. Die Instrumente müssen so nicht verändert werden und die bekannten, gespeicherten geometrische Forminformationen werden zur Erkennung der 3D-Form des Instruments und schließlich der Bestimmung der Lage des Instruments verwendet.

Vorzugsweise kann das Trackingsystem ein Infrarotbasiertes Kamerasystem und/oder elektromagnetisch-basiertes System und/oder ein IMU (Inertial Measurement Unit)-basiertes Verfolgungssystem aufweisen. Im Falle einer IMU (Intertialeinheit) kann diese insbesondere in dem Aufnahmekopf angeordnet sein, um den Aufnahmekopf nachzuverfolgen.

Die Aufgaben der vorliegenden Offenbarung werden hinsichtlich eines medizinischen mobilen Navigationsturms dadurch gelöst, dass dieser aufweist: ein Navigationssystem gemäß der vorliegenden Offenbarung; und eine fahrbare Basis/ fahrbaren Wagen mit Rädern für eine mobile Platzierung des Navigationsturms. Durch die Ausgestaltung als kompakte mobile Einheit kann der Navigationsturm flexibel an unterschiedlichen Stellen in einem Operationssaal platziert werden. Es wird also ein medizinischer mobiler und rollbarer Wagen mit dem Navigationssystem gemäß der vorliegenden Offenbarung vorgeschlagen, der insbesondere einen Computer, welcher die Steuereinheit implementiert, und einen Monitor aufweist.
Hinsichtlich eines Navigationsverfahren zur Navigation bei einem chirurgischen Eingriff bei einem Patienten zur Nachverfolgung/ Tracken von zumindest einem medizinischen Instrument, insbesondere bei einem chirurgischen Navigationssystem der vorliegenden Offenbarung, werden die Aufgaben gelöst durch die Schritte: Registrieren eines Teilabschnitts eines Patienten, insbesondere des Patienten, gegenüber 3D-Aufnahmedaten des Patienten;
Erfassen und Nachverfolgen des nachzuverfolgenden Instruments durch einen Aufnahmekopf einer medizinischen, bildgebenden Vorrichtung;
Erfassen und Nachverfolgen des Aufnahmekopfs der medizinischen, bildgebenden Vorrichtung durch ein Trackingsystem;
Bestimmen/ Berechnen einer Position und/oder Orientierung des medizinischen Instruments durch Verknüpfung der Nachverfolgung des Aufnahmekopfs und der Nachverfolgung des medizinischen Instruments;
Insbesondere Übertragen der erfassten Position und/oder Orientierung des medizinischen Instruments auf die 3D-Aufnahmedaten (3DA); und
Ausgeben einer Kombinationsdarstellung der 3D-Aufnahmedaten mit zumindest der Position und/oder Orientierung des medizinischen Instruments durch eine Darstellungsvorrichtung.

Gemäß einer Ausführungsform kann das Navigationsverfahren ferner die Schritte aufweisen: Erstellen einer Stereo-Aufnahme durch die bildgebende medizinische Vorrichtung; Erstellen, auf Basis der Stereo-Aufnahme, einer Tiefenkarte mit Tiefeninformationen durch eine Bildverarbeitungstechnik und/oder durch eine Triangulation und/oder durch eine Rekonstruktion einer Disparitätsüberlappung; und Bestimmen der Position und/oder Orientierung des Instruments auf Basis der Stereo-Aufnahme und der Tiefenkarte.

Insbesondere kann das Navigationsverfahren, im Falle, dass das vorbestimmte optische Muster ein QR-Code ist und dem QR-Code ein Abstand von dem QR-Code zu der Instrumentenspitze enkodiert vorliegt, ferner die Schritte aufweisen: Dekodieren des QR-Codes; Auslesen des Abstandes zu der Instrumentenspitze; Bestimmen der Position der Instrumentenspitze gegenüber dem Aufnahmekopf und über den nachverfolgen Aufnahmekopf gegenüber den 3D-Aufnahmedaten.

Hinsichtlich eines computerlesbares Speichermedium werden die Aufgaben dadurch erfüllt, indem dieses Befehle umfasst, die bei einer Ausführung durch einen Computer diesen veranlassen, die Verfahrensschritte des Navigationsverfahrens gemäß der vorliegenden Ausführungsform auszuführen.

Jegliche Offenbarung im Zusammenhang mit dem Navigationssystem der vorliegenden Offenbarung gilt ebenso für das Navigationsverfahren der vorliegenden Offenbarung wie auch umgekehrt.

### Kurzbeschreibung der Figuren

Die vorliegende Erfindung wird nachfolgend anhand bevorzugter Ausführungsbeispiele unter Bezugnahme auf die begleitenden Figuren näher erläutert. Es zeigen:
Fig. 1 eine schematische Ansicht eines chirurgischen Navigationssystems einer bevorzugten Ausführungsform mit einem Stereo-Operationsmikroskop;
Fig. 2 eine detaillierte schematische Teilansicht des Navigationssystems aus Fig. 1 mit Nachverfolgung des Instruments durch einen Aufnahmekopf des Operationsmikroskops;
Fig. 3 eine schematische Ansicht der Stereo-Aufnahme des Mikroskopkopfs aus Fign. 1 und 2, in denen ein chirurgisches Instrument erfasst ist;
Fig. 4 eine schematische Ansicht einer 3D-Rekonstruktion durch Triangulation der Stereo-Aufnahme aus Fig. 1 bis 3;
Fig. 5 eine schematische Ansicht einer ermittelten Tiefenkarte der Stereo-aufnahme für eine dreidimensionale Erfassung des nachzuverfolgenden Instruments;
Fig. 6 ein chirurgisches Navigationssystem einer weiteren, zweiten bevorzugten Ausführungsform, bei dem an einem distalen Endabschnitt des Instruments ein QR-Code sichtbar angebracht ist;
Fig. 7 ein chirurgisches Navigationssystem einer weiteren, dritten bevorzugten Ausführungsform, bei dem an einem distalen Endabschnitt des Instruments beabstandete Ring-Gruppen angebracht und sichtbar sind;
Fig. 8 einen mobilen Navigationsturm mit einem chirurgischen Navigationssystem einer weiteren, vierten bevorzugten Ausführungsform; und
Fig. 9 ein Flussdiagramm eines Navigationsverfahrens einer bevorzugten Ausführungsform.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

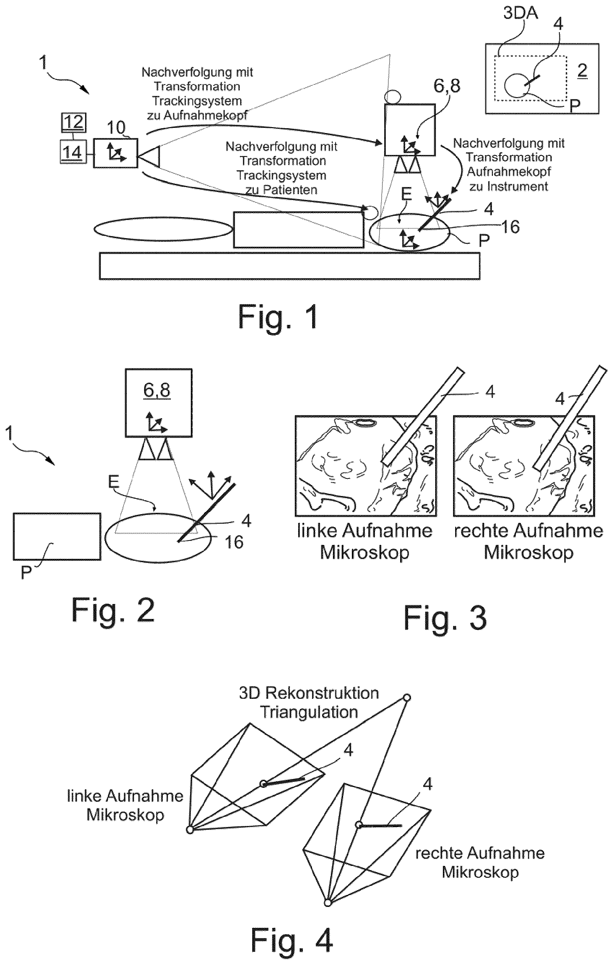

Fig.1 zeigt, zur Erläuterung der vorliegenden Offenbarung und dessen Prinzipien, in einer schematischen Seitenansicht ein chirurgisches Navigationssystem 1 zur Navigation bei einem chirurgischen Eingriff bei einem Patienten P. Das Navigationssystem 1 weist für eine visuelle Ausgabe der Navigationsinformationen an den Chirurgen eine Darstellungsvorrichtung 2 in Form eines OP-Monitors auf.

In einem Eingriffsbereich des Patienten, hier an einem Kopf des Patienten, agiert der Chirurg mit einem medizinischen Instrument 4, beispielsweise einer Pinzette, einem Saugrohr oder einem Versiegelungsinstrument, das er mit dem Navigationssystem 1 nachverfolgen möchte, und führt den Eingriff entsprechend durch.

Ein Stereo-Operationsmikroskop (nachstehend nur Mikroskop genannt) 6 als medizinische bildgebende Vorrichtung weist dabei einen (Stereo-)Mikroskopkopf als Aufnahmekopf 8 auf, der über ein optisches System (nicht dargestellt) mit nachgeschalteten Sensoren wie CMOS-Sensoren oder CCD-Sensoren verfügt, um bei dem Patienten P eine visuelle Stereo-Aufnahme A (zwei zueinander beabstandete Videoaufnahmen aus unterschiedlichen Positionen) eines Teils bzw. Abschnitts des Eingriffsbereichs E zu erstellen. Neben dem Eingriffsbereich selbst erfasst das Mikroskop 6 auch das nachzuverfolgende medizinische Instrument 4, welches in dem Eingriffsbereich E durch den Chirurgen eingesetzt wird. Wie später folgend noch näher erläutert, kann das Navigationssystem 1 auf Basis der Stereo-Aufnahme A das Instrument räumlich in seiner Position und seiner Orientierung, also seiner Lage gegenüber dem Aufnahmekopf 8 erfassen und nachverfolgen bzw. tracken.

Ein externes Trackingsystem 10 des Navigationssystems 1, insbesondere ein infrarotbasiertes Trackingsystem 10, in Form einer externen (Stereo-)Kamera, das im Bereich der Beine des Patienten angeordnet ist, erfasst wiederum den Aufnahmekopf 8 des Mikroskops 6 und kann dieses, insbesondere, wenn an diesem Infrarot-Marker angebracht sind, dreidimensional erfassen und nach verfolgen.

Eine Datenbereitstellungseinheit in Form einer Speichereinheit 12, etwa ein SSD-Speicher, stellt präoperative, digitale 3D-Aufnahmedaten 3DA in Form von MRT-Aufnahmen und/oder CT-Aufnahmen des Patienten P für die Navigation bereit. Der Patienten P wird durch das Trackingsystem 10 erfasst, beispielsweise sind am Kopf des Patienten Infrarot-Marker angeordnet, und gegenüber den 3D-Aufnahmedaten 3DA registriert. Auf diese Weise sind die "realen" zeitaktuellen Daten, insbesondere die Stereo-Aufnahme A, zu den "virtuellen" 3D-Aufnahmedaten 3DA korreliert. Das Trackingsystem 10 erfasst auch während des Eingriffs eine mögliche Bewegung des Körpers des Patienten und registriert bei einer Bewegung den Patienten erneut auf die 3D-Aufnahmedaten 3DA.

Vorliegend ist eine Steuereinheit 14 des Navigationssystem 1 speziell dafür angepasst, die Daten der bildgebenden Vorrichtung 6, die Daten des Trackingsystems 10 sowie die bereitgestellten 3D-Aufnahmedaten 3DA zu verarbeiten und durch eine Verknüpfung der Nachverfolgung von dem Trackingsystem 10 zu dem Aufnahmekopf 8 sowie der Nachverfolgung von dem Aufnahmekopf 8 zu dem Instrument 4 eine Lage des nachzuverfolgenden Instruments 4 und ferner eine Position einer Instrumentenspitze 16 des Instruments 4, zu bestimmen. Konkret stellt die Nachverfolgung des Aufnahmekopfs 8 (Mikroskopkopf) durch das Trackingsystem 10 eine erste Transformationsmatrix bereit, um von dem lokalen Koordinatensystem des Trackingsystems 10, hier einem lokalen Koordinatensystem der externen (Stereo-)Kamera auf das zeitaktuelle lokale Koordinatensystem des Aufnahmekopfs 8 zu schließen. Die Nachverfolgung (tracken) des Instruments 4 durch den Aufnahmekopf 8 stellt wiederum eine zweite Transformationsmatrix von dem lokalen Koordinatensystem des Aufnahmekopfs 8 zu dem lokalen Koordinatensystem des Instruments 4 bereit. Diese erste und zweite Transformationsmatrix verarbeitet die Steuereinheit 14 zu einer Gesamt-Transformationsmatrix von dem lokalen Koordinatensystem der externen (Stereo-)Kamera hin zu dem Instrument 4 selbst.

Zudem wurde ja der Patient P durch das Trackingsystem 10 registriert, also auch eine Relation eines lokalen Koordinatensystems des Patienten P zu dem lokalen Koordinatensystem der externen (Stereo-)Kamera erfasst, welche der Steuereinheit 14 als patientenseitige Transformationsmatrix bereitgestellt wird. Zudem ist der Patient P zu den 3D-Aufnahmedaten 3DA registriert, so dass (idealerweise) der reale Patient P zu den 3D-Aufnahmedaten 3DA übereinstimmt.

Durch einerseits die Gesamt-Transformationsmatrix von dem Instrument 4 (über Aufnahmekopf 8) zu der externen Kamera, und weiter von der externen Kamera zu dem Patienten P bzw. zu den 3D-Aufnahmedaten 3DA lässt sich also die Lage (Position und Orientierung) des nachzuverfolgenden Instruments 4 auf die 3D-Aufnahedaten 3DA übertragen und entsprechen für eine Navigation anzeigen. Die Steuereinheit erstellt eine Korrelationsdarstellung, die einerseits die zu dem Patienten P registrierten 3D-Aufnahmedaten 3DA aufweist und andererseits die Position und/oder Orientierung des Instruments 4, insbesondere ein virtuelles geometrisches Modell des Instruments 4, in den 3DA-Aufnahmedaten 3DA (positionskorrekt oder orientierungskorrekt, insbesondere lagekorrekt) mit anzeigt. Diese Korrelationsdarstellung wird dann durch den OP-Monitor ausgegeben und der Chirurg kann zu jeder Zeit sehen, wo sich das Instrument 4 mit seiner Instrumentenspitze 16 im Patienten P befindet, selbst wenn die Instrumentenspitze 16 nicht sichtbar sein sollte.

Durch die Kombination zweier Nachverfolgungen kann dem Chirurgen eine besonders flexible und sichere Navigation zur Hand gegeben werden. Üblicherweise steht ein Sichtbereich des Mikroskops 6 senkrecht auf einen Eingriffsbereich E mit dem Gewebe, so dass eine Verdeckung durch andere Objekte so gut wie nicht stattfindet. Auch können standardisierte nachzuverfolgende Instrumente 4 bei dem Eingriff für die Navigation eingesetzt werden, da aufgrund der guten visuellen Erfassung durch das Mikroskop 6 mit einhergehender Möglichkeit der Nachverfolgung keine speziellen Pointer-Instrumente notwendig sind.

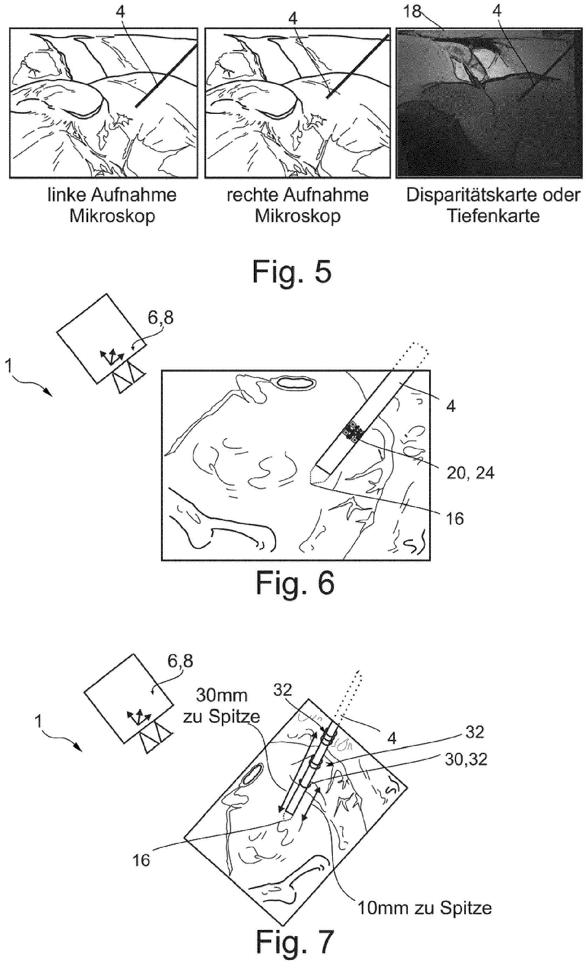

Zur Erläuterung der Nachverfolgung des Instruments 4 durch den Aufnahmekopf 8 zeigen Fign. 2 und 3 einerseits das Mikroskop 6 aus Fig. 1 in detaillierter Teilansicht sowie eine beispielhafte (zweidimensionale) Stereo-Aufnahme A. Der Aufnahmekopf 8 des Mikroskops 6 hat ein optisches System und zwei nachgeschaltete, beabstandete Bildsensoren, die in Folge zwei (leicht) unterschiedliche Aufnahmen (links und rechts) aus den entsprechend unterschiedlichen Perspektiven bereitstellen, wie sie in Fig. 3 schematisch dargestellt sind. Mittels Bildanalyse der linken und rechten Aufnahme kann dann eine Tiefe ermittelt werden, wie nachstehend unter Bezugnahme der Fign. 4 und 5 erläutert wird.

In Fig. 4 ist schematisch das Prinzip einer 3D-Rekonstruktion aus den zwei zweidimensionalen Aufnahmen (Stereo-Aufnahme A mit linker und rechter Aufnahme) erläutert. Hierbei wird durch das Prinzip der Triangulation eine Tiefeninformation für eine dreidimensionale Rekonstruktion ermittelt. So kann mit der Stereo-Aufnahme eine räumliche, dreidimensionale Erfassung erfolgen, insbesondere die dreidimensionale Erfassung des nachzuverfolgenden Instruments 4. Die Nachverfolgung des Instruments 4 wird dabei durch die Steuereinheit 14 ausgeführt. Es reicht also in dieser Ausführungsform, wenn das Operationsmikroskop 6 lediglich die Stereo-Aufnahme A der Steuereinheit 14 bereitstellt, so dass die Steuereinheit 14 hierauf basierend mit der vorstehend beschriebenen Bildanalyse die Lage des Instruments 4 gegenüber dem Aufnahmekopf 8, genauer gesagt zu den Sensoren, ermittelt.

Es kann also mittels der Verknüpfung der Nachverfolgung des Instruments 4 durch den Aufnahmekopf 8 auf Basis einer Bildanalyse und der Nachverfolgung des Aufnahmekopfs 8 durch das Trackingsystem 10 und den zu dem Patienten P registrierten 3D-Aufnahmedaten 3DA in besonders einfacher und sicherer Weise die Lage des Instruments 4, insbesondere die Position der Instrumentenspitze 16, in den 3D-Aufnahmedaten dargestellt werden.

In Fig. 5 ist wieder eine 3D Rekonstruktion auf Basis der Stereo-Aufnahme A gezeigt. Hierbei wird für jeden Pixel in der linken Aufnahme ein korrespondierender Pixel in der rechten Aufnahme gesucht (oder umgekehrt), wobei auf Basis dieser beiden Pixel eine Tiefe des Pixels durch die Steuereinheit 14 berechnet wird. Wenn dies für jeden Pixel der Stereo-Aufnahme A durchgeführt wird, so entsteht schließlich eine Tiefenkarte 18 (schematisch im rechten Bild in Fig. 5 angedeutet), mit der sich, zusammen mit der linken und rechten Aufnahme der Stereo-Aufnahme A, eine räumliche dreidimensionale Struktur bestimmen und so auch das Instrument 4 in seiner Lage und die Position der Instrumentenspitze 16 erfassen lässt.

Fig. 6 zeigt schematisch ein chirurgisches Navigationssystem 1 gemäß einer weiteren, zweiten bevorzugten Ausführungsform mit einer beispielhaften Aufnahme. Das Navigationssystem 1 unterscheidet sich zu dem aus Fign. 1 bis 5 lediglich dadurch, dass dieses vorliegend ein speziell angepasstes nachzuverfolgendes Instrument 4 mit einer optischen Markierung 20 hat und die Steuereinheit 14 dafür angepasst, der optischen Markierung Informationen zuzuordnen und für eine Bestimmung einer Position einer Instrumentenspitze 16 zu verwenden.

Konkret ist in einem distalen Endbereich 22 des Instruments 4 ein QR-Code 24 an einer seitlichen Oberfläche/ Seiten-Oberfläche 26 des Instruments 4 eingraviert. Hierdurch kann etwa ein standardisiertes Instrument 4 nachträglich verändert und angepasst werden, wobei nur eine besonders einfach und schnell herzustellende Eingravierung, beispielsweise mittels Laser, vorgenommen werden muss. In dem QR-Code 24 sind Informationen enkodiert, die die Steuereinheit entschlüsseln und interpretieren kann. Einerseits kann in dem QR-Code 24 direkt ein Abstand in cm enkodiert sein, so dass unabhängig von einem Auswerte-Programm direkt von der Position des QR-Codes 24 entlang einer Längsachse 28 des Instruments 4 mit Betrag des Abstands auf die Position der Instrumentenspitze 16 geschlossen werden kann, oder der QR-Code kann eine ID als Referenz aufweisen, um mittels in der Speichereinheit 12 gespeicherten Daten, welche auch einen Abstand von dem QR-Code 24 zu der Instrumentenspitze 16 haben, auf die Position der Instrumentenspitze 16 zu schließen. Auf diese Weise kann die Instrumentenspitze 16 auch ohne direkten Sichtkontakt in den 3D-Aufnahmedaten 3DA eingeblendet werden, um den Chirurgen bei der Navigation zu unterstützen.

Fig. 7 zeigt eine weitere bevorzugte Ausführungsform eines chirurgischen Navigationssystems. Im Unterschied zu der zweiten Ausführungsform mit dem QR-Code 24 weist das nachzuverfolgende Instrument 4 umfängliche Ringe 30 auf, die in Ring-Gruppen 32 zusammengefasst sind. Die Ring-Gruppen 32 sind entlang der Längsachse 28 des Instruments 4 mit umfänglichen Ringen 30 angeordnet und kodieren einen Abstand von der entsprechenden Ring-Gruppe 32 zu der Instrumentenspitze 16. In 10cm Abstand ist eine erste Ring-Gruppe 32 mit einem einzelnen umfänglichen Ring angeordnet, in 20cm eine zweite Ring-Gruppe 32 mit zwei Ringen, und in 30cm Abstand eine dritte Ring-Gruppe 32 mit drei Ringen 30. Die Steuereinheit 14 ist andererseits dafür angepasst, anhand der Ring-Gruppen 32 den Abstand zu der Instrumentenspitze 16 zu bestimmen.

Mittels der optischen Markierungen 20 an besonders gut einsehbaren Abschnitten des Instruments 4 lässt sich eine Position, insbesondere eine Lage, der zugehörigen Instrumentenspitze 16 gegenüber der optischen Markierung und ggf. auch gegenüber charakteristischen Design-Merkmalen enkodieren oder es lassen sich sogar Geometrieinformationen des Instruments 4 direkt oder über eine Datenbank indirekt enkodieren.

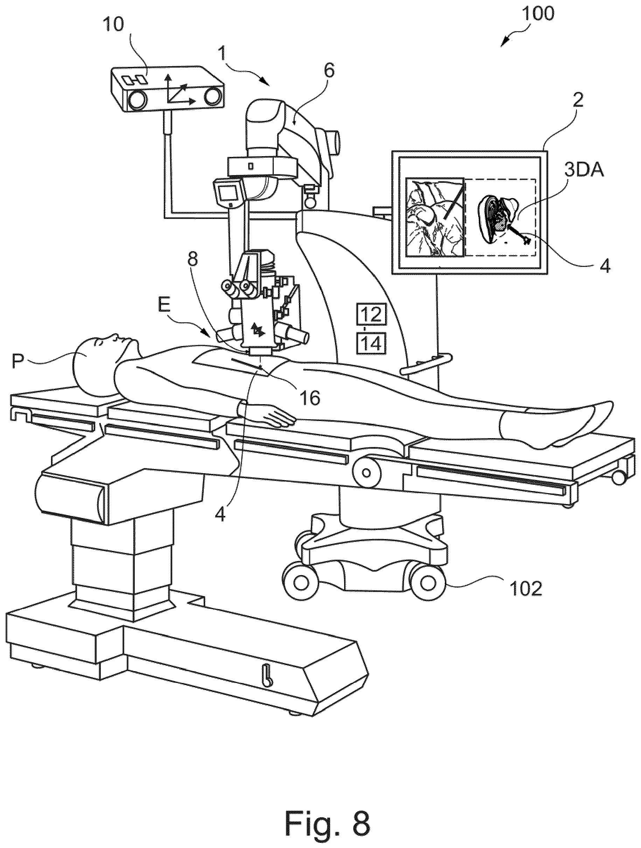

Fig. 8 zeigt einen mobilen Navigationsturm 100 mit einem chirurgischen Navigationssystem 1 einer weiteren, vierten bevorzugten Ausführungsform. Durch die Konfiguration mit Rädern 102 lässt sich der Navigationsturm 100 an unterschiedlichen Orten in einem Operationssaal flexibel einsetzen. Auf dem Monitor kann der Chirurg eine Nebeneinanderdarstellung der Aufnahme des Mikroskops und der Korrelationsdarstellung mit den 3D-Aufnahmedaten 3DA und der eingeblendeten Lage des Instruments 4 anzeigen.

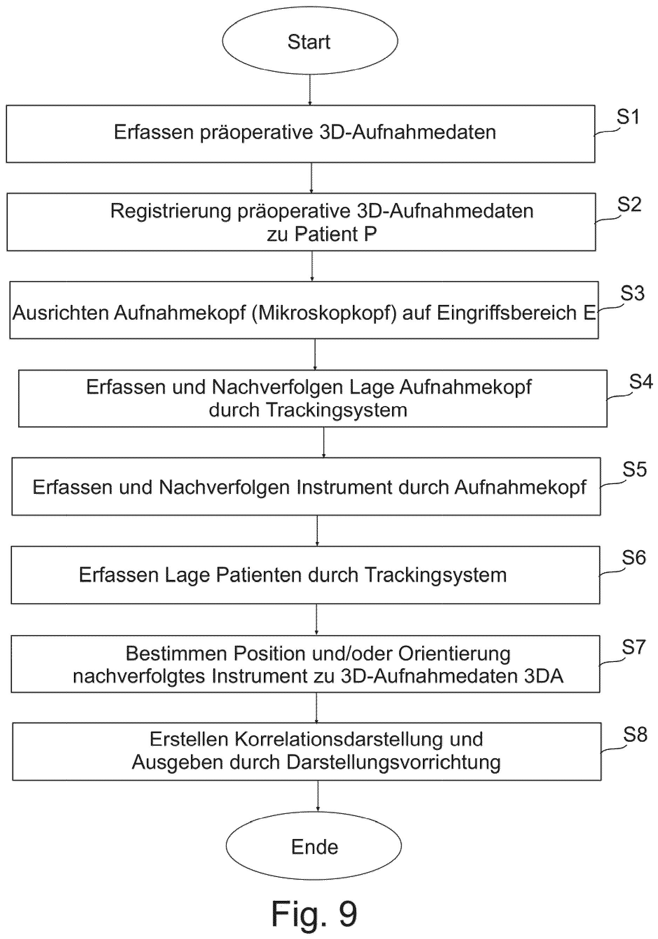

Fig. 9 zeigt in einem Flussdiagramm ein Navigationsverfahren gemäß einer bevorzugten Ausführungsform, welches bei einem chirurgischen Navigationssystem, insbesondere bei einem vorstehend beschriebenen Navigationssystem 1, durchgeführt werden kann.

In einem ersten Schritt S1 werden präoperative 3D-Aufnahmedaten des Patienten P erfasst.

In einem zweiten Schritt S2 wird der Patient P zu den 3D-Aufnahmedaten 3DA registriert.

Vorzugsweise erfolgt in einem Schritt S3 ein Ausrichten der bildgebenden Vorrichtung 6 (Visualisierungssystem) bzw. des Aufnahmekopfs 8 auf den Eingriffsbereich E am Patienten P.

In einem Schritt S4 wird der Aufnahmekopf der bildgebenden Vorrichtung durch das Trackingsystem lokalisiert und nachverfolgt.

In einem Schritt S5 wird eine (dreidimensionale) Lage (Position und Orientierung) des Instruments gegenüber dem Aufnahmekopf 8 über die bildgebende Vorrichtung, insbesondere über die Stereo-Aufnahme, ermittelt.

Vorzugsweise kann in einem Schritt das chirurgische Instrument 4 in dem Eingriffsbereich E von dem Chirurgen geführt werden.

Vorzugsweise wird in einem Schritt S6 der Patient durch das Trackingsystem 10 dreidimensional erfasst und lokalisiert.

In einem nachfolgenden Schritt S7 erfolgt die Berechnung der Position und/oder Orientierung des Instruments 4 relativ zu dem Patienten P und damit zu den zuvor registrierten 3D-Aufnahmedaten 3DA.

Schließlich wird in einem Schritt S8 eine Korrelationsdarstellung erzeugt und die Position und/oder Orientierung des Instruments 4 in den 3D-Aufnahmedaten 3DA eingeblendet und über den Monitor ausgegeben.

### Bezuszeichenlüste

- 1: Chirurgisches Navigationssystem
- 2: Darstellungsvorrichtung
- 4: medizinisches Instrument
- 6: bildgebende Vorrichtung / Stereomikroskop
- 8: Aufnahmekopf
- 10: Trackingsystem
- 12: Speichereinheit
- 14: Steuereinheit
- 16: Instrumentenspitze
- 18: Tiefenkarte
- 20: optische Markierung
- 22: distaler Endbereich des Instruments
- 24: QR-Code
- 26: Seiten-Oberfläche
- 28: Längsachse Instrument
- 30: Ring
- 32: Ringgruppen
- 100: Navigationsturm
- 102: Räder

- P: Patient
- E: Eingriffsbereich
- A: (Stereo-)Aufnahme
- 3DA: 3D Aufnahmedaten

- S1: Schritt Erfassen präoperative 3D-Aufnahmedaten
- S2: Schritt Registrieren Patient zu 3D-Aufnahmedaten
- S3: Schritt Ausrichten Aufnahmekopf auf Eingriffsstelle
- S4: Schritt Nachverfolgen Aufnahmekopf durch Trackingsystem
- S5: Schritt Nachverfolgen Instrument durch Aufnahmekopf
- S6: Schritt Erfassen Lage Patient durch Trackingsystem
- S7: Schritt Bestimmen Position und/oder Orientierung Instrument zu 3D-Aufnahmedaten
- S8: Schritt Erstellen Korrelationsdarstellung und Ausgeben durch Darstellungsvorrichtung

## Patentansprüche

1. Chirurgisches Navigationssystem (1) zur Navigation bei einem chirurgischen Eingriff bei einem Patienten (P) zur Nachverfolgung von zumindest einem medizinischen Instrument, aufweisend:
eine Darstellungsvorrichtung (2), insbesondere einen Monitor, zur Darstellung eines visuellen Inhalts;
zumindest ein nachzuverfolgendes medizinisches Instrument (4);
eine Datenbereitstellungseinheit, insbesondere eine Speichereinheit (12), welche dafür angepasst ist, digitale 3D-Aufnahmedaten (3DA), insbesondere präoperative 3D-Aufnahmedaten (3DA), des Patienten (P) bereitzustellen;
eine medizinische bildgebende Vorrichtung (6) mit einem Aufnahmekopf (8);
ein Trackingsystem (10), welches dafür angepasst ist, den Aufnahmekopf (8) der bildgebenden Vorrichtung (6) zu erfassen und nachzuverfolgen, sowie zumindest einen Teilabschnitt des Patienten (P) für eine Registrierung zu den 3D-Aufnahmedaten (3DA) zu erfassen und insbesondere nachzuverfolgen;
**gekennzeichnet dadurch dass**, der Aufnahmekopf der medizinischen bildgebenden Vorrichtung (6) dafür angepasst ist, bei dem Patienten (P) eine Aufnahme eines Abschnitts eines Eingriffsbereichs (E) zu erstellen sowie das nachzuverfolgende medizinische Instrument (4) zu erfassen und gegenüber dem Aufnahmekopf (8) nachzuverfolgen; und durch
eine Steuereinheit (14), die dafür angepasst ist, die Daten der bildgebenden Vorrichtung (6), die Daten des Trackingsystems (10) sowie die bereitgestellten 3D-Aufnahmedaten (3DA) zu verarbeiten und durch Verknüpfung der Nachverfolgung von dem Trackingsystem (10) zu dem Aufnahmekopf (8) sowie der Nachverfolgung von dem Aufnahmekopf (8) zu dem Instrument (4) eine Position und/oder Orientierung des nachzuverfolgenden Instruments (4), insbesondere einer Instrumentenspitze (16) des Instruments (4), zu bestimmen und eine Korrelationsdarstellung mit den zu dem Patienten (P) registrierten 3D-Aufnahmedaten (3DA) und der Position und/oder Orientierung des Instruments (4), insbesondere der Instrumentenspitze (16), zu erstellen und durch die Darstellungsvorrichtung (2) auszugeben.

2. Chirurgisches Navigationssystem (1) nach Anspruch 1, **dadurch gekennzeichnet, dass** die medizinische bildgebende Vorrichtung (6) ein chirurgisches Operationsmikroskop oder ein medizinisches Endoskop ist, das dafür angepasst ist, eine dreidimensionale Erfassung zur Nachverfolgung durchzuführen, insbesondere ein 3D-Kamerasystem zur Erfassung von Tiefeninformationen aufweist.

3. Chirurgisches Navigationssystem (1) nach Anspruch 1 oder 2, **dadurch gekennzeichnet, dass** der Aufnahmekopf (8) der medizinischen bildgebenden Vorrichtung (6) eine Stereo-Kamera für eine Stereo-Aufnahme (A) aufweist und insbesondere die Steuereinheit (14) dafür angepasst ist, mittels maschinellen Sehens aus der Stereo-Aufnahme (A) eine Position und/oder Orientierung des Instruments (4), insbesondere der Instrumentenspitze (16), gegenüber dem Aufnahmekopf (8) zu erfassen.

4. Chirurgisches Navigationssystem (1) nach Anspruch 3, **dadurch gekennzeichnet, dass** die Steuereinheit (14) dafür angepasst ist, die Position und/oder Orientierung des Instruments (4) mittels Triangulation der Stereo-Aufnahme (A) und/oder mittels Rekonstruktion einer Disparitätsüberlappung der Stereo-Aufnahme (A) zu bestimmen.

5. Chirurgisches Navigationssystem (1) nach einem der vorstehenden Ansprüche, **dadurch gekennzeichnet, dass** an einer Außenseite des nachzuverfolgenden medizinischen Instruments (4), insbesondere an einem distalen Endabschnitt des Instruments (4), ein vorbestimmtes optisches Muster (20), insbesondere ein QR-Code (24) und/oder ein Barcode und/oder zwei zueinander beabstandete Ringe (30), angeordnet, insbesondere in das Instrument (4) integriert, ist, und die Steuereinheit (14) dafür angepasst ist, das optische Muster (20) zu entschlüsseln oder mit einer in einer Speichereinheit hinterlegten Referenz zu vergleichen und auf Basis des erfassten optischen Musters (20) eine Position einer Instrumentenspitze (16) relativ zu dem optischen Muster (20) oder eine Geometrie des Instruments (4) zu bestimmen.

6. Chirurgisches Navigationssystem (1) nach einem der vorstehenden Ansprüche, **dadurch gekennzeichnet, dass** in einer Speichereinheit (12) eine geometrische Form des zumindest einen medizinischen Instruments (4), insbesondere von mehreren medizinischen Instrumenten, gespeichert ist, insbesondere durch eine initiale dreidimensionale Erfassung über den Aufnahmekopf (8) und/oder das Trackingsystem (10), und die Steuereinheit (14) auf Basis eines durch den Aufnahmekopf (8) erfassten Teilabschnitts des medizinischen Instruments (4) und der gespeicherten geometrischen Form die Position, insbesondere Lage, der Instrumentenspitze (16) bestimmt.

7. Chirurgisches Navigationssystem (1) nach einem der vorstehenden Ansprüche, **dadurch gekennzeichnet, dass** das Trackingsystem (10) ein infrarotbasiertes Kamerasystem und/oder elektromagnetisch-basiertes System und/oder ein IMU basiertes Verfolgungssystem aufweist.

8. Chirurgisches Navigationssystem (1) nach einem der vorstehenden Ansprüche, **dadurch gekennzeichnet, dass** das Navigationssystem (1) eine Bildanalyseeinrichtung aufweist, die dafür angepasst ist, mittels maschinellen Sehens aus zumindest zwei Aufnahmeperspektiven, insbesondere einer Stereo-Aufnahme (A), eine räumliche dreidimensionale Erfassung einer Lage eines Instruments (4) durchzuführen.

9. Medizinischer mobiler Navigationsturm (100), aufweisend:
ein chirurgisches Navigationssystem (1) nach einem der vorstehenden Ansprüche; und
einen fahrbaren Wagen mit Rädern (102) für eine mobile Platzierung des Navigationsturms (100).

10. Navigationsverfahren zur Nachverfolgung von zumindest einem medizinischen Instrument (4), insbesondere bei einem chirurgischen Navigationssystem (1) nach einem der vorstehenden Ansprüche, bestehend aus den folgenden Schritten:
Registrieren (S2) eines Teilabschnitts eines Patienten (P), insbesondere des Patienten (P), gegenüber 3D-Aufnahmedaten (3DA) des Patienten (P);
Erfassen und Nachverfolgen (S5) des nachzuverfolgenden medizinischen Instruments (4) durch einen Aufnahmekopf (8) einer medizinischen bildgebenden Vorrichtung (6);
Erfassen und Nachverfolgen (S4) des Aufnahmekopfs (8) durch ein Trackingsystem (10);
Bestimmen (S7) einer Position und/oder Orientierung des medizinischen Instruments (4), insbesondere einer Instrumentenspitze (16), durch Verknüpfung der Nachverfolgung des Aufnahmekopfs (8) und der Nachverfolgung des medizinischen Instruments (4);
Vorzugsweise Übertragen der bestimmten Position und/oder Orientierung des medizinischen Instruments (4) auf die 3D-Aufnahmedaten (3DA); und
Ausgeben (S8) einer Korrelationsdarstellung mit den 3D-Aufnahmedaten (3DA) und mit der Position und/oder Orientierung des medizinischen Instruments (4) durch eine Darstellungsvorrichtung (2).

11. Navigationsverfahren nach Anspruch 10, weiterhin umfassend die folgenden Schritte:
Erstellen einer Stereo-Aufnahme (A) durch die bildgebende medizinische Vorrichtung (6);
Erstellen, auf Basis der der Stereo-Aufnahme (A), einer Tiefenkarte (18) mit Tiefeninformationen durch eine Triangulation und/oder durch Rekonstruktion einer Disparitätsüberlappung; und
Bestimmen der Position und/oder Orientierung des Instruments (4) auf Basis der Stereo-Aufnahme (A) und der Tiefenkarte (18).

12. Navigationsverfahren nach Anspruch 10 oder 11, weiterhin umfassend die folgenden Schritte:
Erfassen eines vorbestimmten optischen Musters (20), insbesondere eines QR-Codes (24) und/oder zumindest zwei zueinander beabstandeter Ringe (30) als Informationsträger für einen Abstand von dem optischen Muster (20) zu einer Instrumentenspitze (16) oder für eine Geometrie des Instruments (4); und
Bestimmen der Position, insbesondere Lage, der Instrumentenspitze (16) relativ zu dem optischen Muster (20) auf Basis des optischen Musters (20).

13. Navigationsverfahren nach Anspruch 12, wobei, im Falle, dass das vorbestimmte optische Muster ein QR-Code (24) ist und in dem QR-Code (24) ein Abstand von dem QR-Code (24) zu der Instrumentenspitze (16) enkodiert vorliegt, das Navigationsverfahren die Schritte aufweist:
Decodieren des QR-Codes (24);
Auslesen des Abstandes zu der Instrumentenspitze (16);
Bestimmen der Position der Instrumentenspitze (16) gegenüber dem Aufnahmekopf (8) und über den nachverfolgen Aufnahmekopf (8) gegenüber den 3D-Aufnahmedaten (3DA).

14. Computerlesbares Speichermedium, das Befehle umfasst, die bei einer Ausführung durch einen Computer diesen veranlassen, die Verfahrensschritte des Navigationsverfahrens gemäß einem der Ansprüche 10 bis 13 auszuführen.

## Claims

1. A surgical navigation system (1) for navigation during a surgical intervention on a patient (P) for tracking of at least one medical instrument, comprising:
a display device (2), in particular a monitor, for displaying visual content; at least one medical instrument to be tracked (4);
a data provision unit, in particular a storage unit (12), which is adapted to provide digital 3D imaging data (3DA), in particular preoperative 3D imaging data (3DA), of the patient (P);
a medical imaging device (6) having an imaging head (8);
a tracking system (10), which is adapted to detect and track the imaging head (8) of the imaging device (6) as well as to detect and in particular track at least a partial portion of the patient (P) for registration to the 3D imaging data (3DA); **characterized in that**
the imaging head of the medical imaging device (6) is adapted to create an image of a portion of an intervention region (E) of the patient (P) as well as to detect and to track the medical instrument (4) to be tracked with respect to the imaging head (8);
a control unit (14), which is adapted to process the data of the imaging device (6), the data of the tracking system (10) as well as the provided 3D imaging data (3DA) and to determine a position and/or orientation of the instrument to be tracked (4), in particular an instrument tip (16) of the instrument (4), by linking the tracking from the tracking system (10) to the imaging head (8) as well as the tracking from the imaging head (8) to the instrument (4) and to create a correlation representation with the 3D imaging data (3DA) registered for the patient (P) and the position and/or orientation of the instrument (4), in particular the instrument tip (16), and to output this by the display device (2).

2. The surgical navigation system (1) according to claim 1, **characterized in that** the medical imaging device (6) is a surgical microscope for surgery or a medical endoscope adapted to perform three-dimensional detection for tracking, in particular comprising a 3D-camera system for detecting depth information.

3. The surgical navigation system (1) according to claim 1 or 2, **characterized in that** the imaging head (8) of the medical imaging device (6) comprises a stereo camera for a stereo image (A) and, in particular, the control unit (14) is adapted to detect a position and/or orientation of the instrument (4), in particular the instrument tip (16), relative to the imaging head (8) from the stereo image (A) via machine vision.

4. The surgical navigation system (1) according to claim 3, **characterized in that** the control unit (14) is adapted to determine the position and/or orientation of the instrument (4) via triangulation of the stereo image (A) and/or via reconstruction of a disparity overlap of the stereo image (A).

5. The surgical navigation system (1) according to one of the preceding claims, **characterized in that** a pre-determined optical pattern (20), in particular a QR code (24) and/or a barcode and/or two rings (30) spaced apart from each other, is arranged on an outer side of the medical instrument (4) to be tracked, in particular on a distal end portion of the instrument (4), in particular integrated into the instrument (4), and the control unit (14) is adapted to decode the optical pattern (20) or to compare it with a reference stored in a storage unit and to determine a position of an instrument tip (16) relative to the optical pattern (20) or a geometry of the instrument (4) on the basis of the detected optical pattern (20).

6. The surgical navigation system (1) according to one of the preceding claims, **characterized in that** a geometric shape of the at least one medical instrument (4), in particular of several medical instruments, is stored in a storage unit (12), in particular by an initial three-dimensional detection by the imaging head (8) and/or by the tracking system (10), and the control unit (14) determines the position, in particular pose, of the instrument tip (16) on the basis of a partial portion of the medical instrument (4) detected by the imaging head (8) and the stored geometric form.

7. The surgical navigation system (1) according to one of the preceding claims, **characterized in that** the tracking system (10) comprises an infrared-based camera system and/or electromagnetic-based system and/or an IMU-based tracking system.

8. The surgical navigation system (1) according to one of the preceding claims, **characterized in that** the navigation system (1) comprises an image analysis device, which is adapted to perform a spatial three-dimensional detection of a pose of an instrument (4) from at least two image perspectives, in particular a stereo image (A) via machine vision.

9. A mobile medical navigation tower (100), comprising:
a surgical navigation system (1) according to one of the preceding claims; and
a mobile cart with wheels (102) for mobile placement of the navigation tower (100).

10. A navigation method for tracking of at least one medical instrument (4), in particular in a surgical navigation system (1) according to one of the preceding claims, consisting of the following steps of:
registering (S2) a partial portion of a patient (P), in particular of the patient (P), with respect to 3D imaging data (3DA) of the patient (P);
detecting and tracking (S5) the medical instrument (4) to be tracked by an imaging head (8) of a medical imaging device (6);
detecting and tracking (S4) the imaging head (8) by a tracking system (10);
determining (S7) a position and/or orientation of the medical instrument (4), in particular an instrument tip (16), by linking the tracking of the imaging head (8) and the tracking of the medical instrument (4);
preferably transferring the determined position and/or orientation of the medical instrument (4) to the 3D imaging data (3DA); and
outputting (S8) a correlation representation with the 3D imaging data (3DA) and with the position and/or orientation of the medical instrument (4) by a display device (2).

11. The navigation method according to claim 10, further comprising the following steps of:
creating a stereo image (A) by the medical imaging device (6);
creating, based on the stereo image (A), a depth map (18) with depth information by triangulation and/or by reconstruction of a disparity overlap; and
determining the position and/or orientation of the instrument (4) based on the stereo image (A) and the depth map (18).

12. The navigation method according to claim 10 or 11, further comprising the following steps of:
detecting a pre-determined optical pattern (20), in particular a QR code (24) and/or at least two rings (30) spaced apart from each other as information carriers for a distance from the optical pattern (20) to an instrument tip (16) or for a geometry of the instrument (4); and
determining the position, in particular pose, of the instrument tip (16) relative to the optical pattern (20) based on the optical pattern (20).

13. The navigation method according to claim 12, wherein in the case that the pre-determined optical pattern is a QR code (24) and a distance from the QR code (24) to the instrument tip (16) is encoded in the QR code (24), the navigation method comprises the steps of:
decoding the QR code (24);
reading the distance to the instrument tip (16);
determining the position of the instrument tip (16) relative to the imaging head (8) and via the tracked imaging head (8) relative to the 3D imaging data (3DA).

14. A computer-readable storage medium comprising instructions which, when executed by a computer, cause the computer to perform the method steps of the navigation method according to one of claims 10 to 13.

## Revendications

1. Système de navigation chirurgicale (1) pour la navigation lors d'une intervention chirurgicale sur un patient (P) pour le suivi d'au moins un instrument médical, présentant :
un dispositif de représentation (2), en particulier un moniteur, pour la représentation d'un contenu visuel ;
au moins un instrument médical (4) à suivre ;
une unité de fourniture de données, en particulier une unité de mémoire (12) qui est adaptée afin de fournir des données d'enregistrement 3D numériques (3DA), en particulier des données d'enregistrement 3D pré-opérationnelles (3DA), du patient (P) ;
un dispositif d'imagerie médicale (6) avec une tête d'enregistrement (8) ;
un système de suivi (10) qui est adapté pour acquérir et suivre la tête d'enregistrement (8) du dispositif d'imagerie médicale (6), ainsi que pour acquérir et en particulier suivre au moins une section partielle du patient (P) pour un enregistrement des données d'enregistrement 3D (3DA) ;
**caractérisé en ce que** la tête d'enregistrement du dispositif d'imagerie médicale (6) est adaptée pour établir un enregistrement d'une section d'une zone d'intervention (E) chez le patient (P) ainsi que pour saisir l'instrument médical (4) à suivre et le suivre par rapport à la tête d'enregistrement (8) ; et par une unité de commande (14) qui est adaptée pour traiter les données du dispositif d'imagerie médicale (6), les données du système de suivi (10) ainsi que les données d'enregistrement 3D (3DA) fournies et pour déterminer une position et/ou une orientation de l'instrument (4) à suivre en combinant le suivi du système de suivi (10) à la tête d'enregistrement (8) ainsi que le suivi de la tête d'enregistrement (8) à l'instrument (4), en particulier une pointe d'instrument (16) de l'instrument (4) et établir une représentation de corrélation avec les données d'enregistrement 3D (3DA) enregistrées pour le patient (P) et la position et/ou l'orientation de l'instrument (4), en particulier de la pointe d'instrument (16), et à la délivrer en sortie par l'intermédiaire du dispositif de représentation (2).

2. Système de navigation chirurgicale (1) selon la revendication 1, **caractérisé en ce que** le dispositif d'imagerie médicale (6) est un microscope opératoire chirurgical ou un endoscope médical qui est adapté pour réaliser une acquisition tridimensionnelle pour le suivi et présente en particulier un système de caméra 3D pour l'acquisition d'informations de profondeur.

3. Système de navigation chirurgicale (1) selon la revendication 1 ou 2, **caractérisé en ce que** la tête d'enregistrement (8) du dispositif d'imagerie médicale (6) présente une caméra stéréo pour un enregistrement stéréo (A) et en particulier l'unité de commande (14) est adaptée pour acquérir, au moyen d'une vision informatique à partir de l'enregistrement stéréo (A), une position et/ou une orientation de l'instrument (4), en particulier de la pointe d'instrument (16), par rapport à la tête d'enregistrement (8).

4. Système de navigation chirurgicale (1) selon la revendication 3, **caractérisé en ce que** l'unité de commande (14) est adaptée pour déterminer la position et/ou l'orientation de l'instrument (4) au moyen d'une triangulation de l'enregistrement stéréo (A) et/ou au moyen d'une reconstruction d'un chevauchement de disparité de l'enregistrement stéréo (A).

5. Système de navigation chirurgicale (1) selon l'une quelconque des revendications précédentes, **caractérisé en ce qu'**un motif optique prédéterminé (20), en particulier un code QR (24) et/ou un code à barres et/ou deux anneaux (30) espacés l'un de l'autre, est disposé, en particulier intégré dans l'instrument (4), sur un côté extérieur de l'instrument médical (4) à suivre, en particulier sur une section d'extrémité distale de l'instrument (4), et l'unité de commande (14) est adaptée pour déchiffrer le motif optique (20) ou le comparer à une référence stockée dans une unité de stockage et déterminer, sur la base du motif optique (20) acquis, une position d'une pointe d'instrument (16) par rapport au motif optique (20) ou une géométrie de l'instrument (4).

6. Système de navigation chirurgicale (1) selon l'une quelconque des revendications précédentes, **caractérisé en ce qu'**une forme géométrique de l'au moins un instrument médical (4), en particulier de plusieurs instruments médicaux, est stockée dans une unité de stockage (12), en particulier par une acquisition tridimensionnelle initiale via la tête d'enregistrement (8) et/ou le système de suivi (10), et l'unité de commande (14) détermine la position, en particulier l'emplacement, de la pointe d'instrument (16) sur la base d'une section partielle de l'instrument médical (4) acquise par la tête d'enregistrement (8) et de la forme géométrique stockée.

7. Système de navigation chirurgicale (1) selon l'une quelconque des revendications précédentes, **caractérisé en ce que** le système de suivi (10) présente un système de caméra basé sur l'infrarouge et/ou un système basé sur l'électromagnétisme et/ou un système de suivi basé sur l'IMU.

8. Système de navigation chirurgicale (1) selon l'une quelconque des revendications précédentes, **caractérisé en ce que** le système de navigation (1) présente un dispositif d'analyse d'images qui est adapté pour effectuer une acquisition tridimensionnelle spatiale d'un emplacement d'un instrument (4) au moyen d'une vision informatique à partir d'au moins deux perspectives d'enregistrement, en particulier d'un enregistrement stéréo (A).

9. Tour de navigation mobile médicale (100), présentant :
un système de navigation chirurgicale (1) selon l'une quelconque des revendications précédentes ; et
un chariot mobile avec des roues (102) pour un placement mobile de la tour de navigation (100).

10. Procédé de navigation pour le suivi d'au moins un instrument médical (4), en particulier dans un système de navigation chirurgicale (1) selon l'une quelconque des revendications précédentes, consistant en les étapes suivantes :
enregistrement (S2) d'une section partielle d'un patient (P), en particulier du patient (P), par rapport à des données d'enregistrement 3D (3DA) du patient (P) ;
acquisition et suivi (S5) de l'instrument médical (4) à suivre par une tête d'enregistrement (8) d'un dispositif d'imagerie médicale (6) ;
acquisition et suivi (S4) de la tête d'enregistrement (8) par un système de suivi (10) ;
détermination (S7) d'une position et/ou d'une orientation de l'instrument médical (4), en particulier d'une pointe d'instrument (16), en combinant le suivi de la tête d'enregistrement (8) et le suivi de l'instrument médical (4) ;
de préférence transfert de la position et/ou l'orientation déterminée de l'instrument médical (4) aux données d'enregistrement 3D (3DA) ; et
délivrance en sortie (S8) d'une représentation de corrélation avec les données d'enregistrement 3D (3DA) et avec la position et/ou l'orientation de l'instrument médical (4) par un dispositif de présentation (2).

11. Procédé de navigation selon la revendication 10, comprenant en outre les étapes suivantes :
établissement d'un enregistrement stéréo (A) par le dispositif d'imagerie médicale (6) ;
établissement, sur la base de l'enregistrement stéréo (A), d'une carte de profondeur (18) avec des informations de profondeur par l'intermédiaire d'une triangulation et/ou d'une reconstruction d'un chevauchement de disparité ; et
détermination de la position et/ou l'orientation de l'instrument (4) sur la base de l'enregistrement stéréo (A) et de la carte de profondeur (18).

12. Procédé de navigation selon la revendication 10 ou 11, comprenant en outre les étapes suivantes :
acquisition d'un motif optique prédéterminé (20), en particulier d'un code QR (24) et/ou d'au moins deux anneaux (30) espacés l'un de l'autre en tant que support d'information pour une distance entre le motif optique (20) et une pointe d'instrument (16) ou pour une géométrie de l'instrument (4) ; et
détermination de la position, en particulier de l'emplacement, de la pointe d'instrument (16) par rapport au motif optique (20) sur la base du motif optique (20).

13. Procédé de navigation selon la revendication 12, dans lequel, dans le cas où le motif optique prédéterminé est un code QR (24) et où une distance entre le code QR (24) et la pointe d'instrument (16) est encodée dans le code QR (24), le procédé de navigation présente les étapes de :
décodage du code QR (24) ;
lecture de la distance jusqu'à la pointe d'instrument (16) ;
détermination de la position de la pointe d'instrument (16) par rapport à la tête d'enregistrement (8) et, par l'intermédiaire de la tête d'enregistrement (8), par rapport aux données d'enregistrement 3D (3DA).

14. Support de stockage lisible par ordinateur qui comprend des instructions qui, lorsqu'elles sont exécutées par un ordinateur, amènent celui-ci à exécuter les étapes de procédé du procédé de navigation selon l'une quelconque des revendications 10 à 13.
